# EUROPÄISCHE PATENTSCHRIFT

(11) **EP 0 872 279 B1**
(45) Veröffentlichungstag und Bekanntmachung des Hinweises auf die Patenterteilung: **15.06.2005**
(21) Anmeldenummer: 98106864.6
(22) Anmeldetag: 16.04.1998
(51) Int. Cl.: A61B 5/00, B01L 3/02, G01N 1/38

(54) **Dosiervorrichtung zur Abgabe kleiner Flüssigkeitsmengen**
Dosing device for dispensing small amounts of liquid
Dispositif de dosage pour delivrer de petites quantités de liquide

(30) Priorität: 17.04.1997 DE 19716073
(43) Veröffentlichungstag der Anmeldung: 21.10.1998
(73) Patentinhaber: Roche Diagnostics GmbH, 68305 Mannheim (DE)
(72) Erfinder: Fritz, Michael, 68647 Biblis (DE); Macho, Heinz, 64658 Fürth (DE); Rinkel, Helmut, 68623 Lampertheim (DE); Schwab, Jürgen, 68775 Ketsch (DE); Arras, Georg, 64385 Reichelsheim (DE); Oswald, Klaus, 64560 Riedstadt-Crumstadt (DE); Schaffner, Alfred, 64395 Brensbach-Höllerbach (DE)

(56) Entgegenhaltungen:
- DE-A- 3 016 594
- GB-A- 946 967
- US-A- 2 965 255
- US-A- 3 233 785
- US-A- 3 603 156
- US-A- 4 563 104

## Beschreibung

Die vorliegende Erfindung betrifft ein System zur dosierten Abgabe von Probenflüssigkeiten, beinhaltend eine Dosiervorrichtung mit einem zusammendrückbaren Ballon, in dessen Wandung eine end-to-end-Kapillare angebracht ist und der Ballon mindestens eine Öffnung hat, durch die der Innenraum mit dem Außenraum kommuniziert, sowie ein Gefäß mit einem Verschluß, der eine Hülse aufweist, in die die Dosiervorrichtung eingesteckt werden kann, so daß die Öffnung im Ballon durch die Hülse verschlossen wird und die end-to-end-Kapillare in das Gefäßinnere hineinragt.

Die vorliegende Erfindung hat ihr bevorzugtes Anwendungsgebiet in der klinischen Chemie, kann jedoch allgemein dort eingesetzt werden, wo es darum geht, eine vorbestimmte kleine Flüssigkeitsmenge dosiert abzugeben. Insbesondere werden derartige Dosiervorrichtungen bei der Blutuntersuchung zur Bestimmung der Zahl der Leukozyten und Erythrozyten eingesetzt.

Im Stand der Technik ist es für Kapillarblutuntersuchungen ein Standardprozedere, zunächst eine Fingerbeere, das Ohrläppchen oder bei Kleinkindern die Ferse mit einer Lanzette zu punktieren und das austretende Blut mit einer end-to-end-Kapillare aufzunehmen. Die end-to-end-Kapillare wird nachfolgend in ein Gefäß mit Verdünnungsflüssigkeit gegeben, das Blut durch die Verdünnungsflüssigkeit herausgespült und das verdünnte Blut einer Untersuchung, in der Regel einer Mikroskopie, unterworfen, In der WO 96/140 11 wird ein Probenabnahmegerät beschrieben, mit der eine derartige Blutuntersuchung vereinfacht werden kann. Ein verschlossenes Gefäß mit Verdünnungsflüssigkeit wird geöffnet und in die Öffnung ein Halter eingesteckt, in dem sich eine end-to-end-Kapilare befindet. Die end-to end-Kapillare wird an die au Probe herangeführt und nimmt infolge der Kapillarkräfte Probenflüssigkeit auf Über den Halter wird nunmehr eine Kappe geschoben, wobei sich der Innendruck in der Kappe erhöht und die Probenflüssigkeit aus der Kapillare hinaus in das Gefäß mit dem Verdünnungsfluid gedrückt wird. Die in der WO 96/140 17 beschriebene Vorrichtung und Vorgehensweise besitzt in der Praxis gravierende Nachteile wie in dem Dokument selbst ausgeführt wird. Es muß bei Benutzung dieser Vorrichtung dafür Sorge getragen werden, daß das Ende der Kapillare, das in die Küvette mit der Verdünnungsflüssigkeit hineinragt, die Flüssigkeit nicht berührt, da sich sonst die Kapillare mit der Verdünnungsflüssigkeit statt der Probe füllen würde. Daher muß die beschriebene Vorrichtung vor Benutzung zusammengesteckt werden, d. h. der Benutzer muß zunächst die Küvette mit dem Verdünnungsfluid öffnen und den Halter einsetzen. Doch auch die Handhabung der bereits zusammengesteckten Vorrichtung ist problematisch, da der Benutzer Acht geben muß, die Anordnungen nicht zu kippen oder zu schütteln, um einen Kontakt zwischen Flüssigkeit und Kapillare zu vermeiden Infolgedessen ist es erforderlich, die Probenflüssigkeit von oben an das freie Ende der Kapillare heranzuführen. Während dies mit einem am Finger befindlichen Blutstropfen noch verhältnismäßig leicht möglich ist, treten, bei der Zuführung anderer Probenflüssigkeiten in der Regel schwerwiegende Handhabungsprobleme auf.

US 2,965,255 beschreibt eine Pipettier- und Dosiervorrichtung, welche eine kapillare Röhre besitzt, welche mit einem elastischen Saugball verbunden, welcher eine Belüftungsöffnung aufweist, über welche durch Abdecken mit einem Finger und Zusammendrücken des Saugballs ein Druckunterschied erzeugt werden kann. Des Weiteren beschreibt US 2,965,255 ein Verdünnungssystem, welches die genannte Pipettiervorrichtung und einen Behälter enthält, welcher ein definiertes Volumen einer Verdünnungslösung enthält. Die Pipettiervorrichtung und der Behälter sind vor der Durchführung des Verdünnungsschritts durch ein röhrenförmiges Verschlussteil getrennt. Dieses Verschlussteil dient primär der Halterung und Lagerung der einzelnen Bestandteile des Verdünnungssystems und der Vermeidung von Verdunstung der Verdünnungsflüssigkeit. Die Entfernung dieses Verschlussteiles erfolgt dadurch, dass nach Entnahme der Pipettiervorrichtung das Verschlussteil durch Herausziehen des gesamten Verschlussteiles vom Behälter weg geschieht.

Aufgabe der vorliegenden Erfindung war es daher, eine Dosiervorrichtung zur Abgabe kleiner Flüssigkeitsmengen zur Verfügung zu stellen, die leicht handhabbar ist und eine hohe Bediensicherheit aufweist.

Die Erfindung wird im Anspruch 1 definiert.

Die vorliegende Erfindung betrifft eine Dosiervorrichtung mit einem zusammendrückbaren Ballon, in dessen Wandung eine end-to-end-Kapilare so angebracht ist, daß ihr eines Ende mit dem Innenraum des Ballons und das andere Ende mit dem Außenraum kommuniziert. Der Ballon besitzt weiterhin mindestens eine Öffnung, über die der Balloninnenraum mit dem Außenraum kommunizieren kann. Diese Öffnung ist so bemessen, daß beim Aufnehmen von Flüssigkeit in die Kapillare Luft aus dem Innenraum des Ballons entweichen kann und somit kein Überdruck entsteht. Hierzu muß die Öffnung einen ausreichend großen Querschnitt besitzen, damit ihr Strömungswiderstand nicht zu groß ist.

In der Praxis hat es sich bewährt Dosierballons mit einem Volumen von 50 µl bis 20 ml einzusetzen und Kapillare zu verwenden, die ein Volumen zwischen 1 und 200 µl aufweisen. Typische Innendurchmesser für die Kapillare liegen zwischen 0,1 und 4 mm. Unter den vorstehend genannten Voraussetzungen liegt der Querschnitt der Öffnung im Ballon vorzugsweise zwischen 0,01 und 3 mm². Der zusammendrückbare Ballon der Dosiervorrichtung kann beispielsweise aus Gummi, einem Gewebe oder einer Metallfolie gefertigt sein. Vorzugsweise ist der Ballon jedoch aus einen Kunststoff wie beispielsweise Polyethylen oder Polypropylen, hergestellt. Bei Verwendung geeigneter Kunststoffe kann der Ballon in einem Spritzgußverfahren oder einem Extrusionsverfahren hergestellt werden. Zur Aufnahme der Kapillare kann bereits während des Herstellprozesses eine Öffnung oder ein Kanal eingebracht werden, in den die Kapillare eingesetzt wird. In gleicher Weise kann auch die mindestens eine Öffnung bereits während des Herstellungsprozesses in den Ballon eingebracht werden. Andererseits ist es auch möglich den Herstellungsprozeß so zu führen, daß eine end-to-end-Kapillare mit einem Kunststoff umspritzt wird. Schließlich kann auch zunächst der Ballon hergestellt und eine Bohrung durch seine Wandung gemacht werden, in die die Kapillare eingesetzt wird. Entsprechend kann auch die Öffnung mit der der Balloninnenraum mit dem Außenraum kommuniziert, nachträglich, beispielsweise mit einem Bohrer oder mit einer Nadel, eingebracht werden.

Zur Vereinfachung der Handhabung sowie zur Verbesserung der Dosiergenauigkeit hat es sich als vorteilhaft erwiesen, im Ballon ein Sichtfenster anzubringen, durch das das ins Balloninnere hineinragende Ende der Kapillare sichtbar ist und so vom Benutzer erkannt werden kann, ob die Kapillare vollständig mit Probeflüssigkeit gefüllt ist. Zur Vermeidung eines Parallaxenfehlers befindet sich das Sichtfenster vorzugsweise auf Höhe des Kapillarendes. Als Sichtfenster kann die ohnehin notwendige Öffnung im Ballon verwendet werden. Günstig ist es auch, für das Sichtfenster einen klaren Kunststoff beispielsweise in Form eines umlaufenden Ringes zu verwenden, der gewölbt ist und somit die Funktion eines Vergrößerungsglases übernimmt.

Es hat sich als vorteilhaft erwiesen, die Dosiervorrichtung mit einer Halterung auszustatten, deren Handhabung keinen Einfluß auf den Druck im Balloninneren hat. Zur Aufnahme von Probenflüssigkeit in die Kapillare wird die Dosiervorrichtung zunächst an diesem Halter gehalten, so daß ein versehentliches Zusammendrücken des Ballons während des Aufnahmeprozesses vermieden werden kann. Die Halterung ist vorzugsweise einstückig mit dem Ballon ausgeführt und kann bereits in dem Spritzguß- oder Extrusionsverfahren des Ballons mit angebracht werden.

Ein Verfahren zur Dosierung von Flüssigkeiten mit der vorstehend geschilderten Dosiervorrichtung verläuft in den folgenden Schritten:
- Heranführen der Kapillare an eine Probenflüssigkeit, wobei sich die Kapillare mit Probenflüssigkeit füllt,
- Abgabe der in der Kapillare befindlichen Probenflüssigkeit durch Zusammendrücken des Ballons

Beim Eintauchen der Kapillare in die Probenflüssigkeit haftet in der Regel auch Probenflüssigkeit an der Außenseite der Kapillare. Es ist daher für eine exakte Dosierung vorteilhaft, die Kapillare vor Abgabe der Probenflüssigkeit abzuwischen. Insbesondere ist dies von Bedeutung, wenn die Außenseite der Kapillare später mit einem Verdünnungsfluid in Kontakt gebracht wird.

Zur Erzielung einer exakten Dosierung ist es weiterhin von Vorteil, die Kapillare mit Verdünnungsfluid zu spülen. In der Regel erfolgt dies, während die Kapillare in das Verdünnungsfluid eintaucht. Durch das wiederholte Aufnehmen von Fluid aus dem Behältnis in die Kapillare und Zurückgeben in das Behältnis kann eine effiziente Spülung erreicht werden.

Die Erfindung umfaßt weiterhin ein System zur dosierten Abgabe von Probenflüssigkeit mit der vorstehend beschriebenen Dosiervorrichtung. Bei diesem System wird die Vorrichtung so in die Hülse eines Gefäßes eingesteckt, daß die Öffnung im Ballon durch die Hülse verschlossen wird und die end-to-end-Kapillare in das Gefäßinnere hineinragt. Die Öffnung muß lediglich ein Entweichen von Luft aus dem Balloninneren ermöglichen. Die Öffnung kann jedoch relativ groß sein, da sie durch das Einstecken in eine Hülse verschlossen wird und die Generierung eines Strömungswiderstandes durch eine begrenzte Größe der Öffnung nicht notwendig ist.

Das Gefäß zur Aufnahme der Dosiervorrichtung besitzt eine Hülse, durch die das Gefäßinnere zugänglich ist. Die Hülse kann mit dem Gefäß einteilig ausgeführt sein oder die Hülse kann ihrerseits in eine Öffnung im Gefäß eingesteckt werden. Die Hülse und der Bereich des Ballons, in dem sich die Öffnung zum Gasaustritt befindet, sind so aneinander angepaßt, daß die Öffnung beim Einstecken des Ballons in die Hülse verschlossen wird. Dies kann auf effektive Weise durch eine Hülse erreicht werden, die in Richtung auf das Gefäßinnere konisch zuläuft. Der Ballon besitzt bei Verwendung einer derartigen Hülse in einem Teilbereich ebenfalls eine konisch zulaufende Gestalt. Um eine sichere Abdichtung der Öffnung zu gewährleisten, sollte der Bereich des Ballons, in dem die Abdichtung erfolgt, möglichst formstabil sein. Dies bedeutet, daß der Ballon vorzugsweise eine erste Region aufweist, die dünnwandig ausgeführt ist, um ein Zusammendrücken auf einfache Weise zu ermöglichen, und einen zweiten Bereich, der eine größere Materialstärke aufweist und schwerer deformierbar ist.

Bei einem System, das eine Einsteckhülse aufweist, in die direkt die Dosiervorrichtung eingesteckt werden kann, besitzt die Einsteckhülse zusätzlich einen Verschluß (z. B. Klapp-, Schraubverschluß oder abziehbare Siegelfolie) oder eine durchstechbare Membran, die den Innenraum des Behältnisses verschließt. Hiermit kann eine Kontamination des Gefäßinneren verhindert werden. Insbesondere bei Gefäßen, die mit einer Verdünnungsflüssigkeit gefüllt sind, ist dies erforderlich, um ein Auslaufen der Flüssigkeit zu verhindern.

Bei einer besonders bevorzugten Ausführungsform der Erfindung besitzt das Gefäß einen Verschluß mit einem Bereich, der geöffnet werden kann. In diesen Öffnungsbereich wird die Einsteckhülse eingebracht. Vorzugsweise kann die Öffnung in dem Verschluß des Gefäßes durch ein Einstecken der Einsteckhülse, erzeugt werden. Hierzu besitzt das Gefäß einen Aufnahmebereich für die Einsteckhülse der eine Sollbruchstelle aufweist. Der Aufnahmebereich kann beispielsweise eine durchstechbare Membrane oder eine Platte aufweisen, die über eine Sollbruchstelle mit dem Verschluß verbunden ist. Beim Einstecken der Einsteckhülse wird dann die Membran durchstochen oder die Sollbruchstelle aufgebrochen, so daß die Einsteckhülse einen Kanal zwischen dem Gefäßinneren und dem Außenraum eröffnet. Der Aufnahmebereich des Verschlusses für die Einsteckhülse ist vorzugsweise eine Führungshülse, die eine definierte Positionierung der Einsteckhülse im Verschluß gewährleistet. Es ist weiterhin vorteilhaft, wenn die Einsteckhülse und die Dosiervorrichtung Rastnasen oder dergleichen aufweisen, die beim Einstecken der Dosiervorrichtung in die Einsteckhülse einrasten und einen Verbleib der Einsteckhülse an der Dosiervorrichtung gewährleisten, wenn die Dosiervorrichtung von dem Gefäß getrennt wird. Durch diese Ausgestaltung ist es besonders gut möglich, die Dosiervorrichtung auch zur Abgabe des mit der Probenflüssigkeit angereicherten Verdünnungsfluids zu verwenden.

Die vorliegende Erfindung beinhaltet dementsprechend auch ein Verfahren zur Abgabe einer um einen vorgegebenen Faktor verdünnten Probenflüssigkeit mit den folgenden Schritten:
- Heranbringen der Dosiervorrichtung mit dem zugänglichen Ende der end-to-end-Kapillare an die zu dosierende Flüssigkeit, so daß sich die Kapillare mit Flüssigkeit anfüllt,
- Einstecken der Dosiervorrichtung in eine Einsteckhülse, die sich an einem Gefäß mit einer definierten Menge Verdünnungsfluid befindet, in der Weise, daß die Öffnung des Ballons durch die Einsteckhülse verschlossen wird,
- Abgabe der in der Kapillare befindlichen Flüssigkeitsmenge in das Gefäßinnere durch Zusammendrücken des Ballons,
- Vermischen von Verdünnungsfluid und Probenflüssigkeit,
- Aufsaugen von verdünnter Probenflüssigkeit in die Kapillare,
- Abnehmen der Dosiervorrichtung von dem Gefäß, wobei vorzugsweise die Einsteckhülse an der Dosiervorrichtung verbleibt,
- Abgabe von verdünnter Probenflüssigkeit durch Zusammendrücken des Ballons der Dosiervorrichtung.

Die vorliegende Erfindung wird anhand einiger Figuren näher erläutert:
- Figur 1:: Dosiervorrichtung
- Figur 2:: System zur dosierten Abgabe von Probenflüssigkeiten
- Figur 3:: System vor Benutzung
- Figur 4:: Öffnung des Gefäßes
- Figur 5 und 6:: Ansicht der Außenumfangsfläche des Verschlußbereiches vor (Figur 5) und nach (Figur 6) Öffnung des Systems
- Figur 7:: Abgabe von Probenflüssigkeit in das Gefäß
- Figur 8:: Gefäß nach Entfernen der Dosiervorrichtung
- Figur 9:: Abgabe von verdünnter Probenflüssigkeit mit der Dosiervorrichtung
- Figur 10:: Detailansicht von Gefäßverschluß, Einsteckhülse und des mit der Öffnung versehenen Bereiches der Dosiervorrichtung

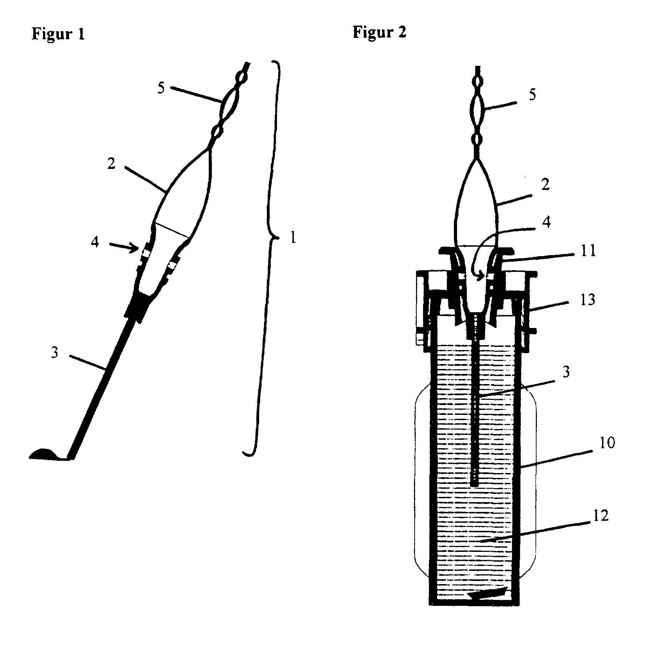

In der Figur 1 ist eine Dosiervorrichtung (1) dargestellt. Die gezeigte Vorrichtung besitzt einen Ballon (2), der in seinem oberen Bereich zusammendrückbar gestaltet ist. In dem unteren Bereich besitzt der Ballon zwei Öffnungen (4). In diesem Bereich ist der Ballon für eine Verwendung in dem nachstehend beschriebenen System vorzugsweise aus stärkerem Material gefertigt als in seinem oberen Bereich, um eine Deformation zu verhindern. In dem unteren Bereich des Ballons befindet sich weiterhin eine Bohrung, in die eine end-to-end-Kapillare (3) eingesteckt ist. Im Anschluß an den oberen Bereich des Ballons befindet sich eine Halterung (5), die zur Handhabung dient. Sofern die Halterung, wie im dargestellten Beispiel, Hohlräume enthält, so wird dafür Sorge getragen, daß diese Hohlräume nicht mit dem Innenraum des Ballons kommunizieren, um Druckschwankungen im Ballon durch eine Handhabung der Halterung zu vermeiden.

In der Figur 2 sind ein System einer Dosiervorrichtung gemäß Figur 1 und ein Gefäß (10) dargestellt, in dem sich eine Flüssigkeit (12) befindet. Das Gefäß besitzt einen Verschluß (13), in dem sich eine Einsteckhülse (11) befindet. Die Dosiervorrichtung ist mit ihrem unteren Bereich so in die Einsteckhülse eingesteckt, daß die Öffnungen (4) verschlossen sind.

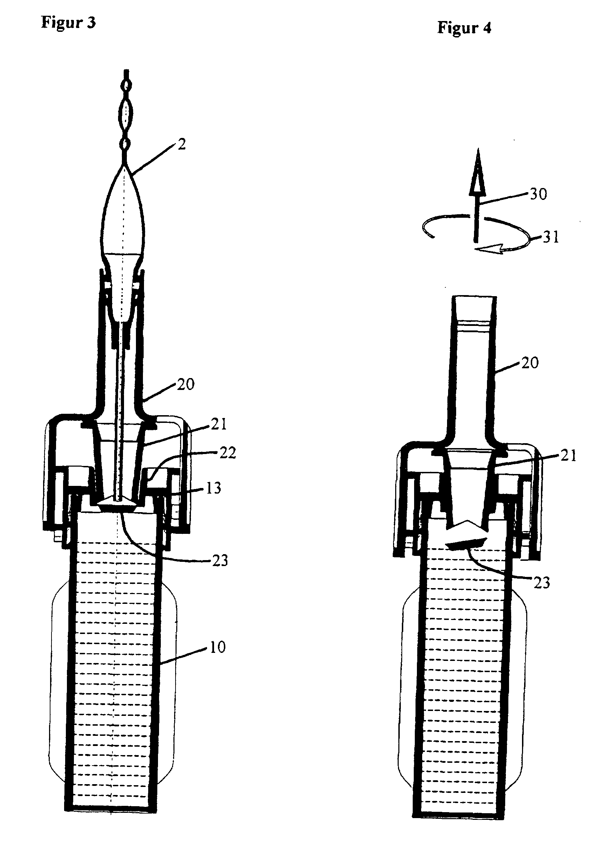

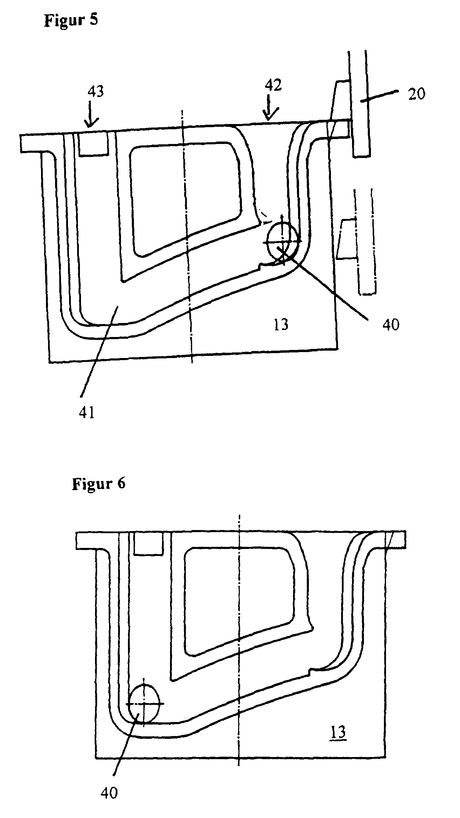

Die nachfolgenden Figuren zeigen ein besonders bevorzugtes System. In der Figur 3 ist dieses System in seinem Ruhezustand vor einer Benutzung dargestellt. Der Ballon (2) ist in einen Zylinder eingesteckt, der sich im oberen Bereich einer Kappe (20) befindet. Die Anordnung ist so gewählt, daß die end-to-end-Kapillare in die Kappe (20) hineinragt und so vor Kontamination und Beschädigung geschützt ist. Die Kappe (20) liegt an einer Einsteckhülse (21) an, die ihrerseits lose in eine Führungshülse (22) eingesteckt ist. Die Führungshülse gehört ihrerseits zu dem Verschluß (13). An der dem Gefäßinneren zugewandten Seite der Führungshülse (22) befindet sich eine Verschlußplatte (23), die mit der Führungshülse (22) über Sollbruchstellen verbunden ist. Die Verschlußplatte (23) stellt einen Bereich des Verschlusses dar, der bei Benutzung des Systems geöffnet werden kann. Wie dies erfolgt, ist in der Figur 4 dargestellt. Vor einer Öffnung des Verschlusses wird zunächst die Dosiervorrichtung aus der Kappe (20) entnommen (siehe Pfeil (30)). Hierauf erfolgt eine Drehung der Kappe (20) gegenüber dem Gefäß (10) (siehe Pfeil (31)). Hierdurch bewegt sich die Kappe (20) in Richtung auf das Gefäß (10) und drückt dabei die Einsteckhülse (21) in Richtung auf das Gefäßinnere, wobei die Verschlußplatte (23) unter Aufbrechen der Sollbruchstellen entfernt wird. Die Einsteckhülse (21) gelangt hierdurch weiterhin in Press-Sitz mit der Führungshülse (22), so daß zwischen Einsteckhülse und Führungshülse kein Gas mehr hindurchtreten kann und somit das Gefäßinnere ausschließlich über die Einsteckhülse (21) mit dem Außenraum kommuniziert. Die Bewegung der Kappe (20) in Richtung auf das Gefäß (10), die durch die Drehung der Kappe vermittelt wird, kann beispielsweise über ein Gewinde realisiert werden. Als besonders vorteilhaft hat sich jedoch hierzu ein Mechanismus erwiesen, der in den Figuren 5 und 6 näher dargestellt ist.

Die Figur 5 korrespondiert zur Figur 3, d. h. zur Position vor Benutzung des Systems. Entsprechend korrespondiert die Figur 6 zur Figur 4. Die Figur 5 zeigt die äußere Umfangsfläche des Verschlusses (13) zusammen mit einem Teil der Kappe (20). An der Innenseite der Kappe (20) befindet sich ein Zapfen (40), der in die Nut (41) auf der Umfangsfläche des Verschlusses eingreift. Die Nut (41) besitzt zwei Öffnungen. Die erste Öffnung (42) ermöglicht es, die Kappe (20) so auf den Verschluß (13) aufzusetzen, daß der Zapfen (40) in die Nut (41) eingreift. Durch Drehen der Kappe (20) gegenüber dem Gefäß und daher auch gegenüber dem Verschluß (13) wird der Zapfen (40) in die in der Figur 6 dargestellte Position gebracht. Durch den schrägen Verlauf der Nut zwischen den dargestellten Positionen des Zapfens findet eine Bewegung der Kappe (20) in Richtung auf das Gefäß (10) statt. Die zweite Öffnung (43) der Nut dient dazu, die Kappe (20) von dem Verschluß (13) abzuziehen, nachdem die Drehung erfolgt ist. Hierdurch wird die Einsteckhülse für ein Einstecken der Dosiervorrichtung zugänglich.

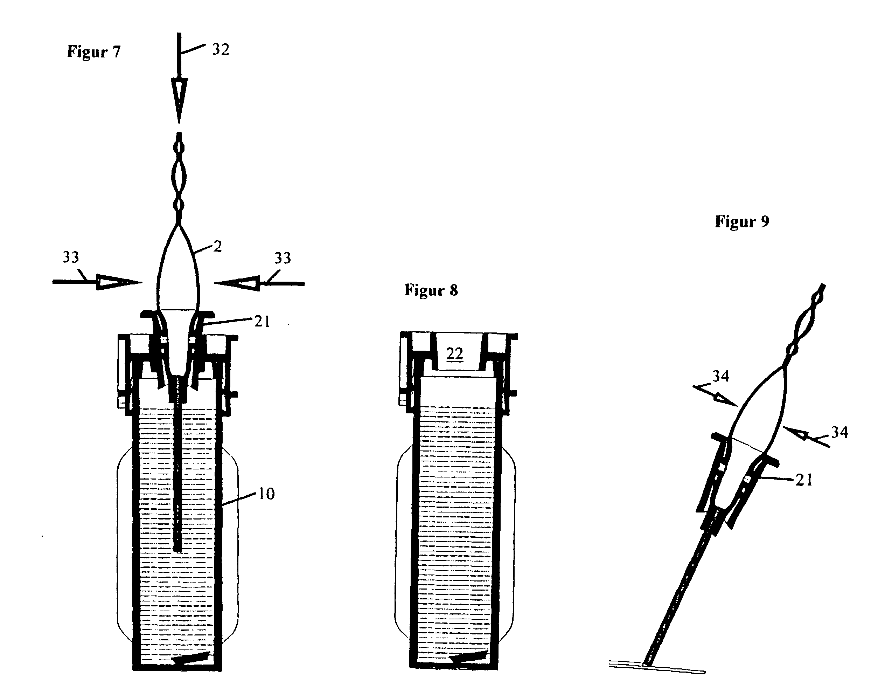

Nachdem die Kappe (20) von dem Verschluß (13) abgezogen wurde, wird die Dosiervorrichtung in die Einsteckhülse (21) eingesteckt, wie dies in der Figur 7 mittels Pfeil (32) dargestellt ist. Bevor jedoch die Dosiervorrichtung eingesteckt wird, erfolgt ein Aufnehmen von Probenflüssigkeit in die Kapillare (3), wie dies bereits in der Figur 1 dargestellt wurde. Nach dem Einstecken wird durch Zusammendrücken des oberen Bereiches des Ballons (2) (siehe Pfeile (33)) die in der Kapillare befindliche Probenflüssigkeit in das Gefäß (10) abgegeben. Wie aus der Figur 7 zu erkennen ist, gelangt der untere Bereich des Ballons mit der Einsteckhülse (21) in Press-Sitz und die in diesem Bereich befindlichen Öffnungen des Ballons werden verschlossen.

Bei einer Entnahme der Dosiervorrichtung aus dem Gefäß (10) verbleibt die Einsteckhülse (21) vorzugsweise an dem Ballon, so daß das Gefäß in der in Figur 8 dargestellten Form zurückbleibt.

Die Dosiervorrichtung mit der daran verbliebenen Einsteckhülse (21) ist in der Figur 9 dargestellt. Durch ein Zusammendrücken des oberen Ballonbereiches (siehe Pfeile (34)) kann verdünnte Probenflüssigkeit auf einen Objektträger oder dergleichen aufgegeben werden.

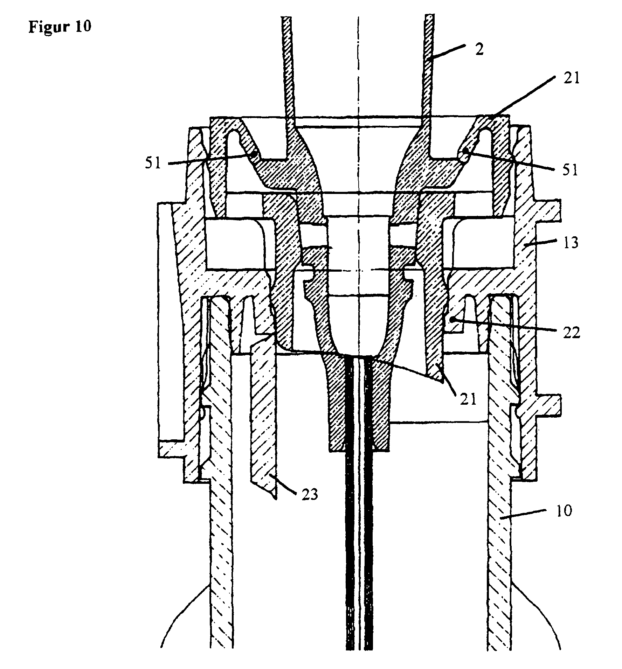

Ein Verbleib der Einsteckhülse (21) an der Dosiervorrichtung kann beispielsweise dadurch realisiert werden, daß die Haftreibung zwischen Dosiervorrichtungen und Einsteckhülse größer ist als zwischen Einsteckhülse und Führungshülse. Vorzugsweise werden jedoch Rastelemente vorgesehen, die sicherstellen, daß die Einsteckhülse zusammen mit der Dosiervorrichtung von dem Gefäß abgezogen wird. Dies ist in der Figur 10 näher dargestellt. Der Ballon (2) besitzt in seinem unteren Bereich einen umlaufenden Wulst (50), der beim Einstecken in die Einsteckhülse (21) an den Rastnasen (51) vorbeigleitet und in der in Figur 10 dargestellten Position einrastet. Durch diese Anordnung ist die Abdichtfunktion der Einsteckhülse für die Öffnungen des Ballons und die Rastfunktion voneinander getrennt. Dementsprechend können beide Funktionen bei der Entwicklung der Dosiervorrichtung getrennt voneinander optimiert werden.

### Bezugszeichenliste

- 1: Dosiervorrichtung
- 2: Ballon
- 3: end-to-end-Kapillare
- 4: Öffnung
- 5: Halterung
- 10: Gefäß
- 11: Hülse
- 12: Flüssigkeit
- 13: Verschluß
- 20: Kappe
- 21: Einsteckhülse
- 22: Führungshülse
- 23: Verschlußplatte
- 30: erster Pfeil
- 31: zweiter Pfeil
- 32: dritter Pfeil
- 33: vierter Pfeil
- 34: fünfter Pfeil
- 40: Zapfen
- 41: Nut
- 42: erste Öffnung der Nut
- 43: zweite Öffnung der Nut
- 50: umlaufender Wulst
- 51: Rastnase

## Patentansprüche

1. System zur dosierten Abgabe von Probenflüssigkeiten, beinhaltend
- eine Dosiervorrichtung (1) mit einem zusammendrückbaren Ballon (2), in dessen Wandung eine end-to-end-Kapillare (3) so angebracht ist, daß das eine Ende der end-to-end-Kapillare (3) mit dem Innenraum des Ballons (2) und das andere Ende mit dem Außenraum kommuniziert und der weiterhin mindestens eine Öffnung (4) hat, durch die der Innenraum des Ballons (2) mit dem Außenraum kommuniziert, und
- ein Gefäß (10) mit einem Verschluß (13), der eine Hülse (11) aufweist, in die der Ballon (2) so eingesteckt werden kann, daß die mindestens eine Öffnung (4) durch die Hülse (11) verschlossen wird und die end-to-end-Kapillare (3) in das Gefäßinnere hineinragt.

2. System gemäß Anspruch 1, bei dem sich im Gefäß (10) eine Flüssigkeit (12) zur Verdünnung von Probenflüssigkeit befindet.

3. System gemäß Anspruch 1, mit einer Dosiervorrichtung (1), die eine Halterung (5) besitzt, deren Handhabung keinen Einfluß auf den Druck im Balloninneren hat.

4. System gemäß Anspruch 1, bei dem das Gefäß (10) einen Verschluß (13) aufweist, welcher einen Bereich aufweist, der geöffnet werden kann und welches eine Einsteckhülse (21) beinhaltet, mit der durch Bewegung in Richtung auf den zu öffnenden Bereich des Verschlusses (13) dieser geöffnet wird und so das Gefäßinnere zugänglich wird.

5. System gemäß Anspruch 4, bei dem der Verschluß (13) eine Führungshülse (22) aufweist, die zur Führung der Einsteckhülse (21) dient.

6. System gemäß Anspruch 4, bei dem der durchstechbare Bereich eine Membran, eine Klappe, ein Stopfen oder ein Bereich mit Sollbruchstelle ist.

7. System gemäß Anspruch 4, bei dem die Dosiervorrichtung (1) so in die Einsteckhülse (21) eingesteckt ist, daß die mindestens eine Öffnung (4) durch die Einsteckhülse (21) verschlossen wird.

8. System gemäß Anspruch 7, bei dem beim Einstecken der Dosiervorrichtung (1) in die Einsteckhülse (21) ein Verklemmen oder Einrasten der Bauteile erfolgt, so daß die Einsteckhülse (21) beim Herausziehen der Dosiervorrichtung (1) aus dem Gefäß (10) an der Dosiervorrichtung (1) verbleibt.

9. System gemäß Anspruch 7, bei dem sich die Öffnung (4) der Dosiervorrichtung (1) in einer Wandung befindet, die beim Einstecken der Dosiervorrichtung (1) in die Einsteckhülse (21) mit der Einsteckhülse (11, 21) in Preßkontakt kommt, so daß die Öffnung (4) durch die Einsteckhülse (11, 21) verschlossen wird.

10. System gemäß Anspruch 4, mit einer Kappe (20), die sich auf dem Verschluß (13) befindet und deren Innenseite so an der Einsteckhülse (21) anliegt, daß die Einsteckhülse (21) durch eine Bewegung der Kappe (20) auf den zu öffnenden Bereich zubewegt wird und ihn öffnet.

11. System gemäß Anspruch 10, bei dem die Kappe (20) an ihrer Innenseite ein Gewinde aufweist, das in ein an dem Verschluß (13) befindliches Gegengewinde eingreift und die Bewegung der Kappe (20) in Richtung auf das Gefäßinnere durch eine Drehung der Kappe (20) erfolgt.

12. System gemäß Anspruch 10, bei dem die Kappe (20) an ihrer Innenseite ein oder mehrere Zapfen (40) aufweist, die in eine Nut (41) in dem Verschluß (13) eingreifen.

13. System gemäß Anspruch 10, bei dem die Kappe (20) an ihrer Innenseite eine Nut besitzt, in die Zapfen, die sich am Verschluß (13) befinden, eingreifen.

14. System gemäß Anspruch 12 oder 13, bei dem die Nut bei Drehung der Kappe (20) gegen über dem Gefäß (10) die ein oder mehreren Zapfen dergestalt führt, daß eine Bewegung der Kappe (20) in Richtung auf den zu öffnenden Bereich erfolgt.

15. System gemäß Anspruch 12, 13 oder 14, mit zwei oder mehr gleichartig geformten Nuten.

16. System gemäß einem der Ansprüche 12 bis 15, bei dem die Nut an einer Stelle geöffnet ist, so daß die Kappe (20) auf den Verschluß (13) aufgesetzt werden kann, wobei die ein oder mehreren Zapfen in die Nut hineingeführt werden.

17. System gemäß Anspruch 16, bei dem die Nut an einer weiteren Stelle geöffnet ist, so daß die Kappe (20), nachdem eine Drehung der Kappe (20) gegenüber dem Gefäß (10) vorgenommen wurde, von dem Verschluß (13) abgezogen werden kann.

18. System gemäß Anspruch 14, bei dem die Nut zwei Schenkel aufweist, die im wesentlichen parallel zur Gefäßachse orientiert sind, und die durch einen Schenkel verbunden sind, der gegenüber der Gefäßachse geneigt ist.

19. System gemäß Anspruch 10, bei dem die Kappe (20) einen Schaft aufweist, in den die Dosiervorrichtung (1) so eingesteckt ist, daß sich die end-to-end-Kapillare (3) vollständig in dem Schaft befindet.

20. Verfahren zur Dosierung von Flüssigkeiten mit einem System gemäß Anspruch 1, mit den Schritten
a) Heranbringen des Endes der end-to-end-Kapillare (3), das mit dem Außenraum kommuniziert, an eine Probenflüssigkeit, so daß sich die Kapillare (3) mit Probenflüssigkeit füllt,
b) Einstecken der Dosiervorrichtung (1) in die Hülse (11), so daß die mindestens eine Öffnung (4) der Dosiervorrichtung (1) durch die Hülse (11) verschlossen wird und die Kapillare (3) in das Gefäßinnere hineinragt,
c) Abgabe der in der Kapillare (3) befindlichen Flüssigkeit in das Gefäß (10) durch Zusammendrücken des Ballons.

21. Verfahren gemäß Anspruch 20, bei dem sich im Gefäß (10) eine Verdünnungsflüssigkeit (12) befindet und nach Schritt c) durch Bewegung des Systems und/oder Spülen der Kapillare (3) eine Durchmischung vorgenommen wird.

22. Verfahren zur Abgabe einer um einen vorgegebenen Faktor verdünnten Probenflüssigkeit mit einem System gemäß Anspruch 1, beinhaltend die Schritte des Verfahrens gemäß Anspruch 20, wobei sich in dem Gefäß eine vorgegebene Menge Verdünnungsflüssigkeit (12) befindet und nach Schritt c) durch Bewegen des Systems und/oder Spülen der Kapillare (3) eine Durchmischung der Flüssigkeiten vorgenommen wird und nachfolgend verdünnte Probenflüssigkeit in die Kapillare (3) aufgesaugt, die Dosiervorrichtung (1) von dem Gefäß (10) abgenommen und durch Zusammendrücken des Ballons (2) in der Kapillare (3) befindliche Flüssigkeit abgegeben wird.

## Claims

1. System for the metered dispensing of sample fluids comprising
- a dispensing device (1) with a compressible balloon (2) in the wall of which an end-to-end capillary (3) is attached in such a manner that one end of the end-to-end capillary (3) communicates with the interior space of the balloon (2) and the other end communicates with the exterior space and additionally has at least one opening (4) through which the interior of the balloon (2) communicates with the exterior, and
- a vessel (10) with a closure (13) which has a collar (11) into which the balloon (2) can be inserted in such a manner that the at least one opening (4) is closed by the collar (11) and the end-to-end capillary (3) projects into the vessel interior.

2. System as claimed in claim 1, in which a fluid (12) for diluting sample fluid is in the vessel (10).

3. System as claimed in claim 1 with a dispensing device (1) which has a holding device (5) whose handling has no effect on the pressure in the interior of the balloon.

4. System as claimed in claim 1, in which the vessel (10) has a closure (13) which has an area that can be opened and which comprises a insertion collar(21) which, when moved towards the area of the closure (13) to be opened, opens this area and thus makes the vessel interior accessible.

5. System as claimed in claim 4, in which the closure (13) has a guide collar (22) which is used to guide the insertion collar (21).

6. System as claimed in claim 4, in which the perforatable section is a membrane, a flap, a stopper or an area with a predetermined breaking point.

7. System as claimed in claim 4, in which the dispensing device (1) is inserted into the insertion collar (21) in such a manner that at least one opening (4) is closed by the insertion collar (21).

8. System as claimed in claim 7, in which insertion of the dispensing device (1) into the insertion collar (21) sticks or locks the components in such a manner that the insertion collar (21) remains on the dispensing device (1) when the dispensing device (1) is removed out of the vessel (10).

9. System as claimed in claim 7, in which the opening (4) of the dispensing device (1) is located in a wall which comes into press contact with the insertion collar (11, 21) when the dispensing device (1) is inserted into the insertion collar (21) in such a manner that the opening (4) is closed by the insertion collar (11, 21).

10. System as claimed in claim 4 with a cap (20) which is located on the closure section (13) and whose inner side rests against the insertion collar (21) in such a manner that the insertion collar (21) is moved by a movement of the cap (20) towards the section to be opened and opens it.

11. System as claimed in claim 10, in which the inner side of the cap (20) has a thread which catches in a counterthread on the closure (13) and the movement of the cap (20) towards the vessel interior is executed by a turning the cap (20).

12. System as claimed in claim 10, in which the inner side of the cap (20) has one or more pegs (40) which catch in a groove (41) in the closure section (13).

13. System as claimed in claim 10, in which the inner side of the cap (20) has a groove in which the pegs located on the closure section (13) match.

14. System as claimed in claim 12 or 13, in which the groove guides the one or more pegs when the cap (20) is turned relative to the vessel (10) in such a manner that the cap (20) moves towards the section to be opened.

15. System as claimed in claim 12, 13 or 14 having two or more grooves having the same shape.

16. System as claimed in one of the claims 12 to 15, in which one site on the groove is open such that the cap (20) can be placed on the closure section (13) thereby inserting the one or more pegs into the groove.

17. System as claimed in claim 16, in which a second site on the groove is open such that the cap (20) can be removed from the closure section (13) after the cap (20) has been turned relative to the vessel (10).

18. System as claimed in claim 14, in which the groove has two branches which are aligned basically parallel to the vessel axis and which are connected by a branch which is diagonal relative to the vessel axis.

19. System as claimed in claim 10, in which the cap (20) has a shaft into which the dispensing device (1) is inserted in such a manner that the end-to-end capillary (3) is entirely enclosed in the shaft.

20. Method for dispensing fluids using a system as claimed in claim 1, comprising the steps
a) bringing the end of the end-to-end capillary (3) which communicates with the exterior space in contact with a sample fluid such that the capillary (3) fills with sample fluid,
b) inserting the dispensing device (1) into the collar (11) such that the at least one opening (4) of the dispensing device (1) is closed by the collar (11) and the capillary (3) extends into the vessel interior,
c) dispensing the fluid in the capillary (3) into the vessel (10) by compressing the balloon.

21. Method as claimed in claim 20, in which a dilution fluid (12) is contained in the vessel (10) and mixing is performed after step c) by moving the system and/or rinsing the capillary (3).

22. Method for dispensing a sample fluid diluted by a predetermined factor using a system as claimed in claim 1, comprising the steps of the method as claimed in claim 20, wherein a prescribed quantity of dilution fluid (12) is contained in the vessel and the fluids are mixed after step c) by moving the system and/or rinsing the capillary (3) and subsequently diluted sample fluid is drawn into the capillary (3), the dispensing device (1) is removed from the vessel (10) and fluid located in the capillary (3) is dispensed by compressing the balloon (2).

## Revendications

1. Système pour la délivrance dosée d'échantillons liquides, contenant
- un dispositif de dosage (1) comprenant un ballon comprimable (2) dans la paroi duquel une pipette capillaire "end to end" (3) est logée de manière à ce qu'une extrémité de la pipette capillaire "end to end" communique avec l'intérieur du ballon (2) et à ce que l'autre extrémité communique avec l'extérieur, lequel ballon est pourvu, en outre, d'au moins une ouverture (4) grâce à laquelle l'intérieur du ballon (2) communique avec l'extérieur et
- un récipient (10) pourvu d'une fermeture (13) comportant une douille (11) dans laquelle le ballon (2) peut être introduit de manière à ce que l'au moins une ouverture (4) soit fermée par la douille (11) et à ce que la pipette capillaire "end to end" (3) entre à l'intérieur du récipient.

2. Système selon la revendication 1, dans lequel un liquide (12) destiné à la dilution d'un échantillon liquide se trouve dans le récipient (10).

3. Système selon la revendication 1 , comprenant un dispositif d e dosage (1) qui est pourvu d'une fixation (5) dont le maniement n'a aucune influence sur la pression à l'intérieur du ballon.

4. Système selon la revendication 1, dans lequel le récipient (10) est pourvu d'une fermeture (13) comportant une zone qui peut être ouverte et est pourvue d'une douille d'introduction (21) qui permet d'ouvrir la zone de la fermeture (13) à ouvrir grâce à un mouvement en direction de celle-ci et rend ainsi accessible l'intérieur du récipient.

5. Système selon la revendication 4, dans lequel la fermeture (13) est pourvue d'une douille de guidage (22) qui sert à guider la douille d'introduction (21).

6. Système selon la revendication 4, dans lequel la zone pouvant être percée est une membrane, un clapet, un bouchon ou une zone pourvue d'un point de rupture.

7. Système selon la revendication 4, dans lequel le dispositif de dosage (1) est introduit dans la douille d'introduction (21) de manière à ce que l'au moins une ouverture (4) soit fermée par la douille d'introduction (21).

8. Système selon la revendication 7, dans lequel, lors de l'introduction du dispositif de dosage (1) dans la douille d'introduction (21), il se produit un coincement ou un encliquetage des éléments de manière à ce que la douille d'introduction (21) reste sur le dispositif de dosage (1) lors du retrait du dispositif de dosage (1) du récipient (10).

9. Système selon la revendication 7, dans lequel l'ouverture (4) du dispositif de dosage (1) se trouve dans une paroi qui entre en contact serré avec la douille d'introduction (11, 21) lors de l'introduction du dispositif de dosage (1) dans la douille d'introduction (21), de manière à ce que l'ouverture (4) soit fermée par la douille d'introduction (11, 21).

10. Système selon la revendication 4, comprenant u n capuchon (20) situé sur la fermeture (13) et dont le côté intérieur est en contact avec la douille d'introduction (21) de manière à ce que la douille d'introduction (21) soit déplacée vers la zone à ouvrir grâce à un mouvement du capuchon (20) et ouvre cette zone.

11. Système selon la revendication 10, dans lequel le capuchon (20) est pourvu, sur son côté intérieur, d'un filet qui se met en prise avec un filet inverse situé sur la fermeture (13) et le mouvement du capuchon (20) en direction de l'intérieur du récipient s'effectue en tournant le capuchon (20).

12. Système selon la revendication 10, dans lequel le capuchon (20) est pourvu, sur son côté intérieur, d'un ou plusieurs tourillon(s) (40) qui s'engage(nt) dans une rainure (41) dans la fermeture (13).

13. Système selon la revendication 10, dans lequel le capuchon (20) est pourvu, sur son côté intérieur, d'une rainure dans laquelle s'engagent des tourillons situés sur la fermeture (13).

14. Système selon la revendication 12 ou 13, dans lequel, lors de la rotation du capuchon (20) par rapport au récipient (10), la rainure guide le ou les tourillon(s) de manière à ce qu'un mouvement du capuchon (20) en direction de la zone à ouvrir ait lieu.

15. Système selon la revendication 12, 13 ou 14, pourvu de deux ou plus de deux rainures de même forme.

16. Système selon une des revendications 12 à 15, dans lequel la rainure est ouverte à un endroit, de manière à ce que le capuchon (20) puisse être posé sur la fermeture (13), le ou les tourillon(s) étant introduits dans la rainure.

17. Système selon la revendication 16, dans lequel la rainure est ouverte à un autre endroit, de manière à ce que le capuchon (20) puisse être enlevé de la fermeture (13) après que l'on a procédé à une rotation du capuchon (20) par rapport au récipient (10).

18. Système selon la revendication 14, dans lequel la rainure comporte deux branches qui sont orientées de manière essentiellement parallèle à l'axe du récipient et qui sont reliées par une branche inclinée par r apport à l'axe du récipient.

19. Système selon la revendication 10, dans lequel le capuchon (20) est pourvu d'une tige dans laquelle le dispositif de dosage (1) est introduit de manière à ce que la pipette capillaire "end to end" (3) soit entièrement contenue dans la tige.

20. Procédé de dosage de liquides avec un système selon la revendication 1, comprenant les étapes :
a) approcher d'un échantillon liquide l'extrémité de la pipette capillaire "end to end" (3) communiquant avec l'extérieur de manière à ce que la pipette capillaire (3) se remplisse d'échantillon liquide,
b) introduire le dispositif de dosage (1) dans la douille (11) de manière à ce que l'au moins une ouverture (4) du dispositif de dosage (1) soit fermée par la douille (11) et à ce que la pipette capillaire (3) entre à l'intérieur du récipient,
c) délivrer le liquide se trouvant dans la pipette capillaire (3) dans le récipient (10) en comprimant le ballon.

21. Procédé selon la revendication 20, dans lequel un liquide de dilution (12) se trouve dans le récipient (10) et on procède à un mélange, après l'étape c), en bougeant le système et/ou en rinçant la pipette capillaire (3).

22. Procédé pour délivrer un échantillon liquide dilué selon un facteur prédéfini à l'aide d'un système selon la revendication 1, comprenant les étapes du procédé selon la revendication 20, une quantité prédéfinie de liquide de dilution (12) se trouvant dans le récipient et un mélange des liquides étant réalisé, après l'étape c), en bougeant le système et/ou en rinçant la pipette capillaire (3), et de l'échantillon liquide dilué étant ensuite aspiré dans la pipette capillaire (3), le dispositif de dosage (1) retiré du récipient (10) et du liquide qui se trouve dans la pipette capillaire (3) délivré en comprimant le ballon (2).
